Europäisches Patentamt

European Patent Office

Office européen des brevets

(11) Publication number:

**0 058 485**
**B1**

## EUROPEAN PATENT SPECIFICATION

(45) Date of publication of patent specification: 24.04.85

(21) Application number: 82300444.5

(22) Date of filing: 28.01.82

(51) Int. Cl.⁴: **A 61 N 1/30, A 61 N 1/04**

(54) Metallised medical treatment electrode with insulated border.

(30) Priority: 12.02.81 US 233899

(43) Date of publication of application:
25.08.82 Bulletin 82/34

(45) Publication of the grant of the patent:
24.04.85 Bulletin 85/17

(84) Designated Contracting States:
AT BE CH DE FR GB IT LI LU NL SE

(56) References cited:
EP-A-0 010 712
FR-A-1 524 246
FR-A-2 452 938
US-A-3 547 105
US-A-4 226 247
US-A-4 247 596

(73) Proprietor: SYBRON CORPORATION
1100 Midtown Tower
Rochester, NY 14604 (US)

(72) Inventor: Behl, Robert S.
85 Matthew Drive
Fairport New York (US)
Inventor: Ellis, Franklin H.
361 Sagamore Drive
Rochester New York (US)

(74) Representative: Oliver, Roy Edward et al
POLLAK MERCER & TENCH High Holborn House
52-54 High Holborn
London WC1V 6RY (GB)

Courier Press, Leamington Spa, England.

## Description

This invention relates to medical electrodes, and more particularly, is concerned with electrodes for use in ion therapy. Many types of wounds or lesions may be treated through the use of a porous metallised treatment electrode energized by a constant current generator. This treatment electrode is typically in the form of a film of an active metallic material such as, silver, which is plated or otherwise coated upon a porous synthetic fibre substrate.

Before use, the treatment electrode size is selected to approximate the shape of the patient's wound or lesion. The electrode is held by gauze or the like so as to contact with the patient's tissue, a return electrode being placed elsewhere on the patient. A tab extends from the electrode to provide electrical contact with a current source. The patient completes an electric circuit formed by the current source, the treatment electrode as an anode, and the return electrode as a cathode. Current flow from the surface of the treatment electrode causes migration of metal ions from the metallic coating in the general direction of the return electrode.

Two problems arise during treatment if the electrode is merely cut to the appropriate size by the manufacturer or by the user.

The first problem is due to loose or protruding fibres from a cut or sheared edge of the electrode which can form a more intimate contact with the tissue than does the remainder of the electrode. This contact provides a lower resistance path with corresponding preferential current and ion flow away from the central portion of the wound or lesion.

The second problem occurs when a moderately conductive solution, such as normal saline, is used to soak the electrode and surrounding tissue for improving conduction and quantity of ion flow. A radial flow of current from the periphery of the treatment area may occur. In both of these situations the diversion of current and ions away from the treatment area has the undesirable effect of reducing the desired ion concentration in this area. Accordingly, it is the primary object of the invention to provide an electrode having an electrically non-conductive and/or solution-impervious border and a method for making the same.

Thus, one aspect of the invention provides an electrode for use in ion therapy comprising a substrate formed of polymeric fibres coated with at least one layer of an electrically-conductive material, characterized by an electrically insulating border extending around at least a portion of the periphery of a treatment area of the electrode, whereby diversion of electric current and ions from the treatment area within the electrically-insulating border is prevented during use of the electrode.

Another aspect of the invention provides a method of fabricating a shaped electrode, for use in ion therapy, from a substrate formed of polymeric fibres coated with at least one layer of an electrically-conductive material, which method comprises applying sufficient heat and pressure around at least a portion of the periphery of the substrate of coated fibres to melt adjacent polymeric fibres and disrupt the electrically-conductive layer material, and to fuse together adjacent fibres, thereby forming an electrically-insulating and solution-impervious border around at least said portion of the periphery of the coated substrate.

US—A—4226247 discloses an electrode comprising conductive fibres extending within a frame, the material of the frame not being specified.

Fig. 1 is a view of a treatment electrode;

Fig. 2 is a cross-sectional view of coated electrode fibres prior to melting;

Fig. 3 shows the fabrication of an electrode border by use of a hand tool to apply heat and pressure sufficient to cause melting;

Fig. 4 illustrates the use of a heated stamp and die for forming a border of melted fibres and cutting an electrode from a web; and

Fig. 5 is a cross-sectional view of fused fibres within a border after melting.

It has been found that the provision of electrically insulating border about at least a portion of the periphery of an ion treatment electrode solves several problems encountered with the prior art electrodes.

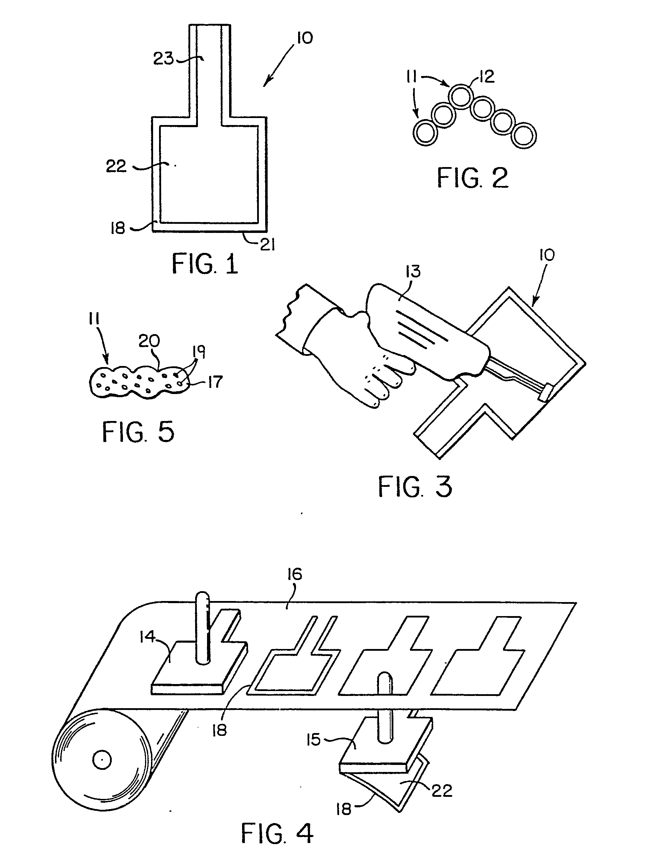

An ion treatment electrode 10, as shown in Fig. 1 of the drawing, is manufactured with a flexible porous substrate or fabric, preferably made of woven or unwoven synthetic polymeric fibres, such as nylon or rayon. As shown in Fig. 2, the fibres 11 are covered with a thin conductive metallic layer 12, preferably of silver, which may be deposited over sub layers of another conductive material, such as tin.

The drawing is not to scale as the total cross-sectional area of the conductive layers has been found to be usually one thousandth or less of the cross-sectional area of a fibre.

In accordance with the invention, the electrode edge is sealed by an electrically insulating border either before or after the electrode is cut. A handheld heat tool 12, such as that illustrated in Fig. 3, can be utilized to make the border after the electrode is hand-cut to shape.

The preferred method, however, is illustrated by Fig. 4. A heated stamp 14, or equivalent means of rapidly applying heat and pressure, is used to melt the adjacent polymer substrate and fuse the fibre. As an additional feature, a die 15, punch, or other cutting means is combined with the heated stamp 14 for simultaneously or sequentially cutting the electrode from a web 16 of surrounding material, while retaining the sealed area as the electrode border.

Fig. 5 shows that the molten polymer 17 disrupts the conductive coating and encapsulates the silver particles 19 and other conductive material. The polymer itself, is an effective electrical insulator, and therefore provides a border 18 with high electrical resistance which prevents measurable current at physiologic voltage levels. The molten polymer fibres fuse together at their junctions 20 at the edge of the fabric substrate. This provides a smooth, non-conductive border 21 on the electrode, as best seen in Fig. 1. The heat bonded fibres are impervious to a conductive solution, such as saline, whilst centre area 22 of the electrode 10 remains conductive and porous.

In order to make electrical contact with the electrode 10, a conductor rail or tab 23 may extend from an edge of the electrode. Preferably, the tab 23 and the remainder of the electrode are integral and cut from the web 16 at the same time. The insulating border 18 is arranged not to cross the tail or tab 23 but to extend along the edge thereof.

## Claims

1. An electrode (10) for use in ion therapy comprising a substrate formed of polymeric fibres (11) coated with at least one layer of an electrically-conductive material (12), characterized by an electrically-insulating border (18) extending around at least a portion of the periphery of a treatment area of the electrode, whereby diversion of electric current and ions from the treatment area within the electrically-insulating border is prevented during use of the electrode.

2. An electrode according to claim 1, which is flexible.

3. An electrode according to claim 1 or 2, which is porous.

4. An electrode according to claim 1, 2 or 3, wherein the treatment area is centrally located with respect thereto.

5. An electrode according to any preceding claim including an electrical connecting tag (23).

6. An electrode according to any preceding claim wherein the electrically-insulating border is impervious to an electrically-conductive solution.

7. An electrode according to any preceding claim, wherein the border has been formed by disruption of the electrical-conductive coating material and melting of the adjacent polymeric substrate fibres.

8. A method of fabricating a shaped electrode, for use in ion therapy, from a substrate formed of polymeric fibres coated with at least one layer of an electrically-conductive material, which method comprises applying sufficient heat and pressure around at least a portion of the periphery of the substrate of coated fibres to melt adjacent polymeric fibres and disrupt the electrically-conductive layer material, and to fuse together adjacent fibres, thereby forming an electrically-insulating and solution-impervious border around at least said portion of the periphery of the coated substrate.

9. A method according to claim 8, wherein the substrate is cut to a desired shape of electrode either before or after the heat and pressure are applied thereto.

10. An electrode when made by a method according to claim 8 or 9.

## Patentansprüche

1. Elektrode (10) für die Ionentherapie mit einem aus polymeren, mit wenigstens einer Schicht aus einem elektrisch leitfähigen Material beschichteten Fasern (11) bestehenden Basismaterial, gekennzeichnet durch einen elektrisch isolierenden Rand (18), der wenigstens einen Teil des Umfangs der Behandlungsfläche der Elektrode (10) umgibt, so daß während des Gebrauchs der Elektrode (10) eine Ablenkung des Stroms und der Ionen aus der durch den elektrisch isolierenden Rand (18) definierten Behandlungszone verhindert wird.

2. Elektrode (10) nach Anspruch 1, gekennzeichnet durch Biegsamkeit.

3. Elektrode (10) nach Anspruch 1 oder 2, gekennzeichnet durch Porosität.

4. Elektrode (10) nach einem der Ansprüche 1 bis 3, dadurch gekennzeichnet, daß die Behandlungsfläche zentral angeordnet ist.

5. Elektrode (10) nach einem der vorangegangenen Ansprüche, gekennzeichnet durch eine elektrische Anschlußlasche (23).

6. Elektrode (10) nach einem der vorangegangenen Ansprüche, dadurch gekennzeichnet, daß der elektrisch isolierende Rand (18) für eine elektrisch leitfähige Lösung undurchlässig ist.

7. Elektrode (10) nach einem der vorangegangenen Ansprüche, dadurch gekennzeichnet, daß der Rand (18) durch Unterbrechung des elektrisch leitfähigen, der Beschichtung dienenden Materials sowie durch Schmelzen der angrenzenden polymeren Fasern des Basismaterials hergestellt ist.

8. Verfahren zur Herstellung einer geformten Elektrode für die Ionentherapie aus einem Basismaterial, das aus polymeren Fasern besteht, die mit wenigstens einer Schicht aus einem elektrisch leitfähigem Material überzogen sind, gekennzeichnet durch die Anwendung von Druck und Wärme entlang wenigstens eines Teils des Umfangs des aus beschichteten Fasern bestehenden Basismaterials, so daß aneinander grenzende polymere Fasern schmelzen, deren jeweilige elektrisch leitfähige Schicht unterbrochen wird und sich aneinander grenzende Fasern miteinander verbinden, wobei wenigstens entlang eines Teils des Umfangs des beschichteten Basismaterials ein elektrisch isolierender und lösungsundurchlässiger Rand gebildet wird.

9. Verfahren nach Anspruch 8, dadurch gekennzeichnet, daß das Basismaterial entweder vor oder nach der Einwirkung von Druck und Wärme zu der gewünschten Gestalt der Elektrode geschnitten wird.

10. Elektrode (10), gekennzeichnet durch die Herstellung durch ein Verfahren gemäß den Ansprüchen 8 und 9.

**Revendications**

1. Electrode (10) utilisable en ionothérapie, comprenant un support formé de fibres de polymère (11) revêtues d'une couche au moins d'une matière électriquement conductrice (12), caractérisée par un bordure électriquement isolante (18) qui s'étend autour d'une partie au moins de la périphérie de la zone de traitement de l'électrode, de manière à empêcher le détournement de courant électrique à partir de la zone de traitement, dans la bordure électriquement isolante, pendant l'utilisation de l'électrode.

2. Electrode selon la revendication 1, caractérisée en ce qu'elle est souple.

3. Electrode selon la revendication 1 ou 2, caractérisée en ce qu'elle est poreuse.

4. Electrode selon l'une quelconque des revendications 1 à 3, caractérisée en ce que la zone de traitement est située au centre par rapport à elle.

5. Electrode selon l'une quelconque des revendications 1 à 4, caractérisée en ce qu'elle comporte une patte de connexion électrique (23).

6. Electrode selon l'une quelconque des revendications 1 à 5, caractérisée en ce que la bordure électriquement isolante est imperméable à une solution électriquement conductrice.

7. Electrode selon l'une quelconque des revendications 1 à 6, caractérisée en ce que la bordure est formée par dislocation de la matière électriquement conductrice de revêtement et par fusion des fibres voisines du support de polymère.

8. Procédé de fabrication d'une électrode façonnée, destinée à être utilisée en ionothérapie, à partir d'un support formé de fibres de polymère revêtues d'une couche au moins d'une matière électriquement conductrice, caractérisé en ce qu'il comprend l'opération consistant à appliquer, autour d'une partie au moins de la périphérie du support de fibres revêtues, une chaleur et une pression suffisantes pour fondre les fibres de polymère voisines et disloquer la matière de la couche électriquement conductrice et pour fusionner des fibres voisines, de manière à former un bordure électriquement isolante et imperméable aux solutions autour de ladite partie au moins de la périphérie du support de fibres revêtues.

9. Procédé selon la revendication 8, caractérisé en ce que le support est découpé à la forme voulue de l'électrode, soit avant soit après l'application de chaleur et de pression à ce support.

10. Electrode fabriquée par un procédé selon la revendication 8 ou 9.

FIG. 1

FIG. 2

FIG. 5

FIG. 3

FIG. 4